# EUROPEAN PATENT SPECIFICATION

(11) **EP 4 477 183 B1**
(45) Date of publication and mention of the grant of the patent: **18.03.2026**
(21) Application number: 24000074.5
(22) Date of filing: 11.06.2024
(51) Int. Cl.: A61B 90/70, A61B 1/12

(54) **ENDOSCOPE WASHING MACHINE WITH DRYING SYSTEM OF THE INTERNAL CHANNELS OF REPROCESSED ENDOSCOPES**
WASCHMASCHINE FÜR ENDOSKOPE MIT INTERNEM KANALTROCKNUNGSSYSTEM VON GENERALÜBERHOLTEN ENDOSKOPEN
MACHINE À LAVER DES ENDOSCOPES AVEC SYSTÈME DE SÉCHAGE INTERNE DES CANAUX POUR DES ENDOSCOPES RECONDITIONNEÉS

(30) Priority: 13.06.2023 IT 202300012144
(43) Date of publication of application: 18.12.2024
(73) Proprietor: Nuova S.B. System S.r.l., 20092 Cinisello Balsamo (MI) (IT)
(72) Inventor: Bongiovanni, Davide, 20092 Cinisello Balsamo (MI) (IT)
(74) Representative: Aprà, Mario

(56) References cited:
- IT-A1- MI20 122 084
- US-A1- 2005 196 314
- US-A1- 2009 044 845
- US-B2- 8 920 574

## Description

The present invention refers to an endoscope washing machine with a drying system of the internal channels of reprocessed endoscopes.

### Background art

Modern endoscope washing machines allow total reprocessing of endoscopic instruments by cleansing and disinfecting the outside and the internal channels of the endoscope. After rinsing the chemical substances utilized to reprocess the endoscope, the standard UNI EN ISO 15883 establishes that it is necessary to dry the internal channels of the endoscope, to limit residues of water inside the instruments, which can also lead to bacterial residues. To this end, known endoscope washing machines allow drying of the internal channels of endoscopes to be carried out using sterile filtered air, normally obtained by means of filters with filter grade of 0.01 µm. This sterile filtered air is, at the current state of the art, obtained with three different methods:
- Using a compressor positioned inside the machine, which supplies compressed air to the endoscope washing machines to dry the internal channels.
- Using a compressor positioned outside the endoscope washing machine, which operates in the same way as the internal compressor, i.e., it supplies compressed air to dry the internal channels of the endoscope.
- Using medical compressed air, which can be supplied by the healthcare structure in which the endoscope washing machine is installed, through suitable medical air inlets installed in wall of the room in which the machine is located.

Endoscope washing machines of the type specified are disclosed in the documents US 2005/196314 A1, IT MI20 122 084 A1 and US 2009/0044845 A1.

However, the drying operation of the internal channels of the endoscope according to the state of the art does not provide for removal of the toxic fumes produced by the use of disinfectant. In particular, known endoscope washing machines do not have a system for the disposal of toxic fumes produced by the use of disinfectant. Therefore, excellent systems are required to ventilate the environment in which the machine is located, i.e., fume absorption systems external to the machine, or rooms with windows with the possibility of external ventilation, in order to create a comfortable and ideal environment that allows operators to work in safe conditions.

### Technical problem underlying the invention

Prior art solutions allow the requirements of drying the internal channels of the endoscope by means of compressors or external medical air coming from the air supply circuits of hospital structures to be met.

The solution that provides for the use of a compressor, although efficient, makes the drying process noisy and, if the compressor is positioned externally, also increases the footprint of all the instrumentation required to reprocess the endoscopes.

Although The solution that provides for the use of external medical air is also efficient for drying the internal channels of the endoscope, it makes the endoscope washing machine dependent on the connection to external medical air, placing an important installation constraint on the machine.

The problem of the disposal of toxic fumes instead limits the installation of endoscope washing machines to rooms that can be well ventilated, i.e., requires the use of suitable filtering systems or exhaust hoods external to the machine, to make the working environment more comfortable for the operators.

As will be more apparent from the description below, the system for drying the internal channels of reprocessed endoscopes in an endoscope washing machine according to the present invention operates without requiring a compressor inside the machine, a compressor installed externally to the machine, or a connection to the external medical air of a healthcare structure. The solution according to the invention allows the air coming from the environment inside the machine, including the toxic fumes produced by the disinfectant and suitably filtered, to be utilized, so as to allow drying of the internal channels of the endoscope treated in the machine and disposal of the toxic residues of the disinfectant.

Therefore, the endoscope washing machine according to the present invention ensures adequate drying without the need for particular installation constraints, making the machine independent from any external sources for the supply and use of sterile air, completely eliminating all the toxic fumes generated by the disinfectant through suitable filtration. In this way, the endoscope washing machine according to the invention can be installed in any type of room inside a healthcare structure, as it does not require particular ventilation conditions.

### Summary of the invention

The invention is defined by the appended independent claim. Further embodiments of the invention are illustrated in the dependent claims.

### Summary of the disclosure

An object of the present disclosure is to provide an endoscope washing machine with a drying system of the internal channels of reprocessed endoscopes, which ensures safe working conditions for the operator in charge of the machine, guaranteeing optimal removal of the acid fumes deriving from the disinfectant.

Another object of the disclosure is to provide an endoscope washing machine with a drying system of the internal channels of reprocessed endoscopes, which ensures complete drying with simple and safe means.

In particular, it is specified that the endoscope washing machine for drying the internal channels of reprocessed endoscopes according to the disclosure comprises a drying system integrated and inside the machine, comprising two different aeraulic circuits:
- a first aeraulic circuit which utilizes the air coming from the washing tank of the endoscope washing machine as purge air, which is pushed into the channels of the endoscope, wherein the purge air is utilized to purge the channels of the treated endoscope internally, removing water to allow the drying air to pass through.
- a second aeraulic circuit, which utilizes the air coming from the internal environment of the endoscope washing machine, also contaminated with fumes generated by the use of peracetic acid, which is first filtered and then pushed into the internal channels of the endoscope to be used as drying air and allow drying of the channels of the endoscope treated.

Further features and advantages of the invention will be more apparent from the detailed description below of an example of embodiment of the machine according to the invention, with reference to the accompanying drawing, which shows important details for the invention, and from the claims.

The features illustrated here must not necessarily be understood as being to scale and are represented so that the characteristics according to the invention are clearly highlighted.

### Description of the drawings

In the drawing:

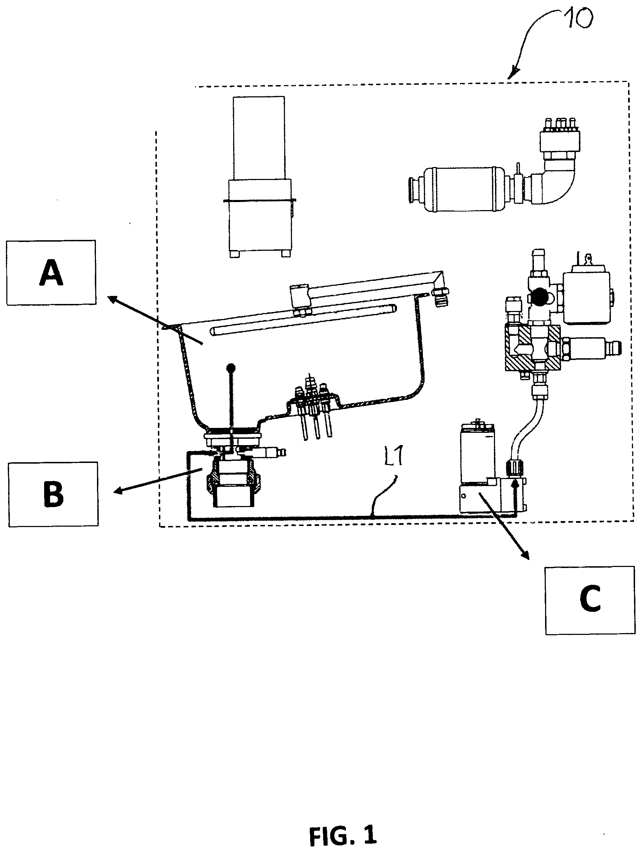

- Figs. 1 to 3 are schematic views, with parts in section, illustrating a part of an endoscope washing machine for drying the internal channels of reprocessed endoscopes according to an example, with reference to the first aeraulic circuit, wherein said Figs. 1 to 3 illustrate respective lengths of said first aeraulic circuit;

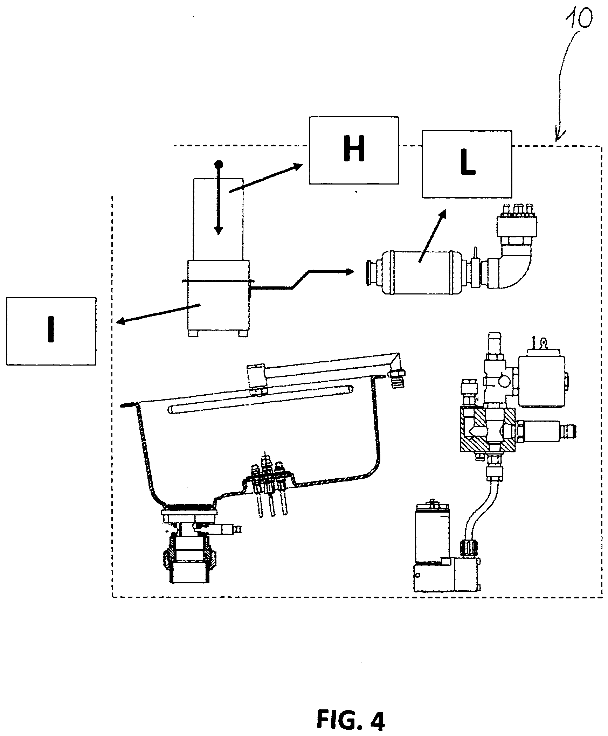

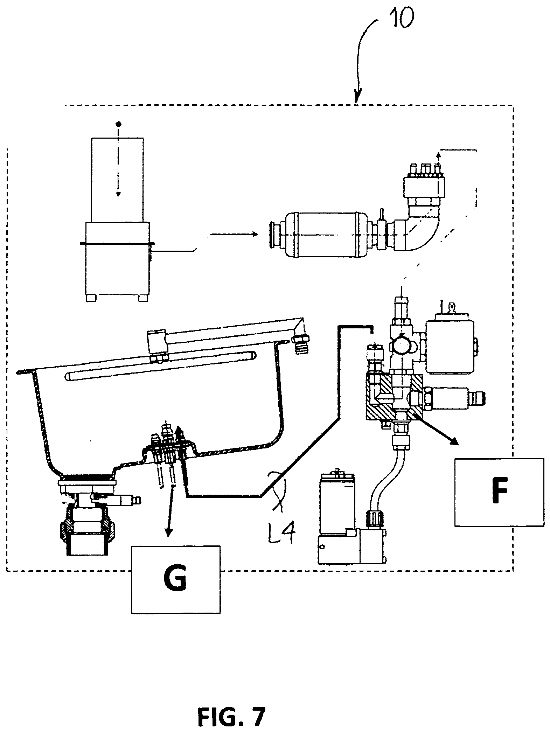

- Figs. 4 to 7 are schematic view, with parts in section, illustrating a part of the endoscope washing machine for drying the internal channels of reprocessed endoscopes according to an example, with reference to the second aeraulic circuit, wherein said Figs. 4 to 7 illustrate respective lengths of said second aeraulic circuit.

### Description of the first aeraulic circuit of the endoscope washing machine (Figs. 1 to 3)

With reference to the drawing, the reference number 10 indicates, schematically, the endoscope washing machine.

The endoscope washing machine 10 comprises a tank A for washing and rinsing an endoscope to be treated (not illustrated). Said tank A has a discharge opening B and the machine 10 comprises a pump C, wherein said pump C has an air intake opening which is pneumatically connected, by means of first pneumatic duct means L1 (Fig. 1), to said discharge opening B of the tank A and collects air from said discharge opening B, after the rinse water has been evacuated from the tank A.

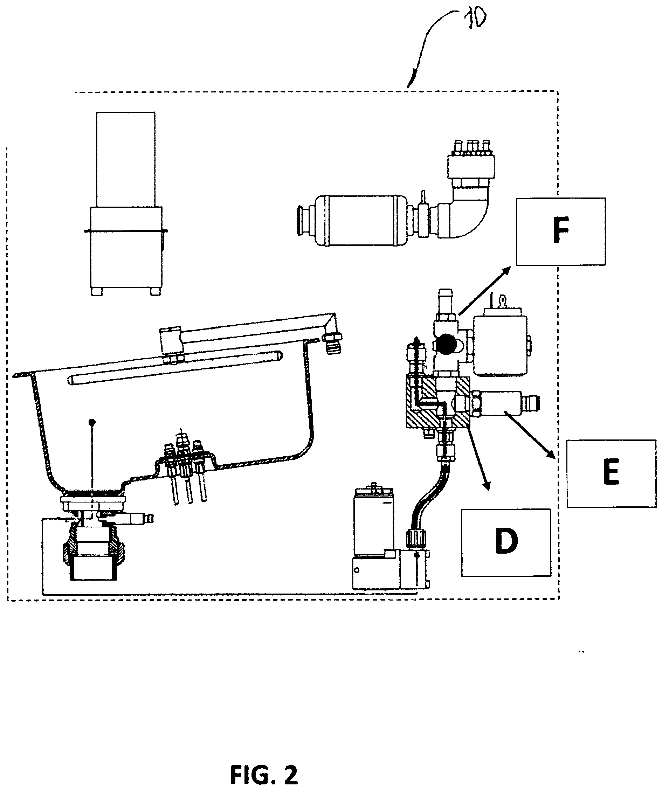

The endoscope washing machine 10 further comprises a manifold D and a solenoid valve F (Fig. 2), wherein said manifold D has an air inlet pneumatically connected to the air delivery outlet of said pump C and a plurality of air outlets, arranged downstream of said air inlet. The machine 10 comprises a solenoid valve F, comprising an air flow diverter that, in a first operating position, channels the compressed air flow from said air inlet of said manifold D to said plurality of air outlets; a pressure sensor E controls the pressure of the air that flows between said inlet and said plurality of air outlets of the manifold D with respect to a preset limit value.

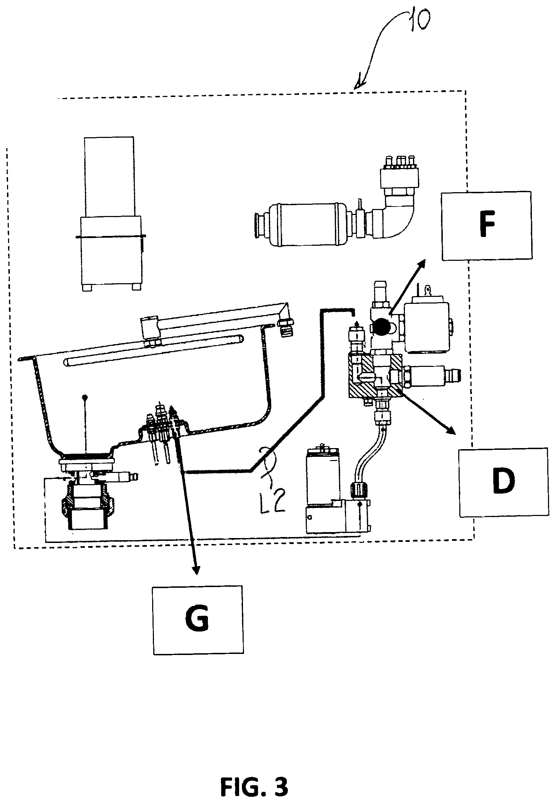

The endoscope washing machine 10 comprises, in the bottom of the tank A, a plurality of pneumatic connectors G (Fig. 3) for corresponding internal channels of the endoscope treated, in which each air outlet of said plurality of air outlets of said manifold D is pneumatically connected, by means of second pneumatic duct means L2, with a respective pneumatic connector of said plurality of pneumatic connectors G.

By means of the aforesaid arrangement, the purge air, collected from the tank A, is utilized to purge the channels of the endoscope treated internally, removing water from the internal circuit of the endoscope treated to allow, in a subsequent step, filtered drying air to pass through.

### Description of the second aeraulic circuit of the endoscope washing machine (Figs. 4 to 7)

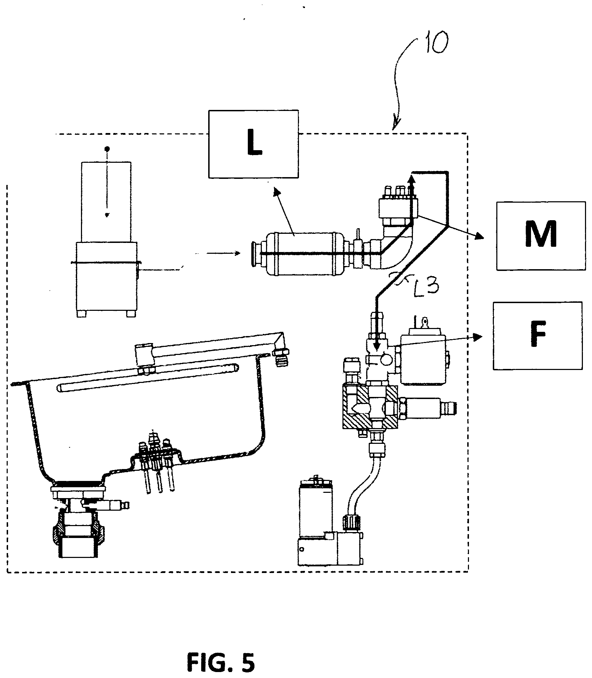

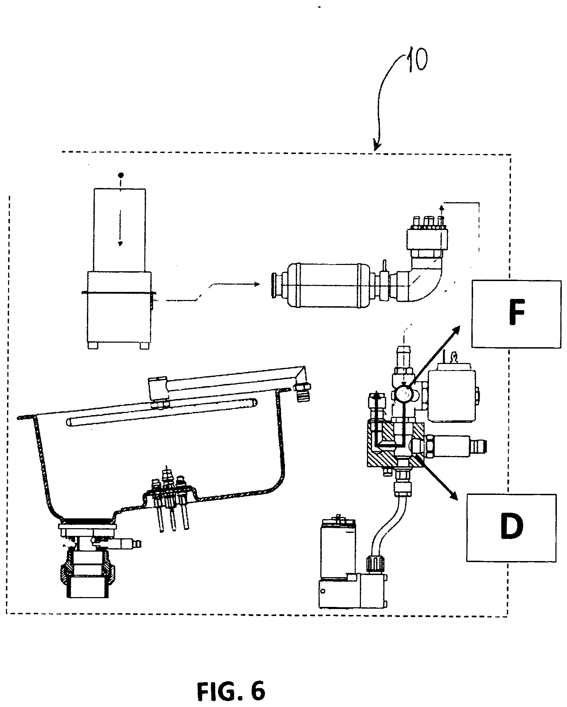

The endoscope washing machine 10 comprises, inside the machine 10 (Fig. 4), a blower device I of internal air collected from the internal circuit of the machine 10, wherein said internal air is blown by the blower device I through a first activated charcoal filter means H, provided inside the machine 10 and configured to remove the toxic fumes of the acid utilized to disinfect the endoscopic instrument arranged inside the tank A from the internal air. A second filter means L, provided inside the machine 10, is arranged downstream of said first filter means H and has a degree of filtration in the order of 0.01 µm. An air inlet of said second filter means L receives the internal air filtered by said first filter means H and, by means of further filtration, makes the internal air sterile. A plurality of sterile air outlets M (Fig.5) of said second filter means L are pneumatically connected, by means of third pneumatic duct means L3, with respective air inlets of said solenoid valve F that, in a second operating position of the respective air flow diverter (Fig. 6), channels the compressed air flow from said respective air inlets of said solenoid valve F to said plurality of air outlets of said manifold D. Said plurality of air outlets of said manifold D are pneumatically connected, by means of fourth pneumatic duct means L4, respectively to said plurality of pneumatic connectors G for corresponding internal channels of the endoscope treated, arranged in the tank A. The filtered internal air is pushed from the manifold D, through the respective pneumatic connectors G, into the internal channels of the endoscope, ensuring optimal drying thereof.

By means of the arrangement described above, the endoscope washing machine 10 guarantees optimal removal of the acid fumes deriving from the disinfectant, making the working environment safe for the operator.

As is apparent from the above, the present disclosure achieves the objects set forth in the introduction of this description, in a simple and efficient way.

## Claims

1. Endoscope washer machine (10) for drying the internal channels of reprocessed endoscopes, **characterized in that** it comprises a drying system integrated and inside the machine, comprising two different aeraulic circuits:
- a first aeraulic circuit (A, B, C, L1, D, E, F, L2, G), in which the air coming from the washing tank (A) of the endoscope washer machine (10) is pushed into the channels of the endoscope to purge the channels of the treated endoscope internally, removing water to allow the drying air to pass through;
- a second aeraulic circuit (I, H, L, M, L3, F, D, G), in which the air coming from the internal environment of the endoscope washer machine (10), also contaminated with fumes generated by the use of peracetic acid, is first filtered through a first activated charcoal filter means (H) and is then pushed into the internal channels of the endoscope to be used as drying air and allow drying of the channels of the endoscope treated.

2. Endoscope washer machine (10) according to claim 1, **characterized in that** said first aeraulic circuit comprises:
- a tank (A) for washing and rinsing an endoscope to be treated, having a discharge opening (B);
- a pump (C), provided with an air intake opening pneumatically connected, by means of first pneumatic duct means (L1), to said discharge opening (B) of the tank (A), and configured to collect air from said discharge opening (B), after the rinse water has been evacuated from the tank (A);
- a manifold (D), having an air inlet pneumatically connected to the air delivery outlet of said pump (C) and a plurality of air outlets, arranged downstream of said air inlet;
- a solenoid valve (F), comprising an air flow diverter that, in a first operating position, channels the compressed air flow from said air inlet of said manifold (D) to said plurality of air outlets of said manifold (D);
- a pressure sensor (E), configured to control the pressure of the air that flows between said inlet and said plurality of air outlets of the manifold (D) with respect to a preset limit value;
- a plurality of pneumatic connectors (G) for corresponding internal channels of the endoscope treated, provided in the bottom of the tank (A), in which each air outlet of said plurality of air outlets of said manifold (D) is pneumatically connected, by means of second pneumatic duct means (L2), with a respective pneumatic connector of said plurality of pneumatic connectors (G), so that the purge air, collected from the tank (A), is utilized to purge the channels of the endoscope treated internally, removing water from the internal circuit of the endoscope treated to allow, in a subsequent step, filtered drying air to pass through.

3. Endoscope washer machine (10) according to claim 1, **characterized in that** said second aeraulic circuit comprises:
- a blower device (I) of internal air collected from the internal circuit of the machine (10), provided inside the machine (10), wherein said internal air is blown by the blower device (I) through a first activated charcoal filter means (H), provided inside the machine (10) and configured to remove the toxic fumes of the acid utilized to disinfect the endoscopic instrument arranged inside the tank (A) from the internal air;
- a second filter means (L), provided inside the machine (10), arranged downstream of said first filter means (H) and having a degree of filtration in the order of 0.01 µm, wherein an air inlet of said second filter means (L) receives the internal air filtered by said first filter means (H) and, by means of further filtration, makes the internal air sterile;
- a plurality of sterile air outlets (M) of said second filter means (L), which are pneumatically connected, by means of third pneumatic duct means (L3), with respective air inlets of said solenoid valve (F) that, in a second operating position of the respective air flow diverter, channels the compressed air flow from said respective air inlets of said solenoid valve (F) to said plurality of air outlets of said manifold (D), wherein:
- said plurality of air outlets of said manifold (D) are pneumatically connected, by means of fourth pneumatic duct means (L4), respectively to said plurality of pneumatic connectors (G) for corresponding internal channels of the endoscope treated, arranged in the tank (A);
- the filtered internal air is pushed from the manifold (D), through the respective pneumatic connectors (G), into the internal channels of the endoscope,
so that the endoscope washer machine (10) dries the channels of the endoscope treated and removes the acid fumes deriving from the disinfectant.

## Patentansprüche

1. Endoskop-Waschmaschine (10) zum Trocknen der inneren Kanäle von aufbereiteten Endoskopen, **dadurch gekennzeichnet, dass** sie ein integriertes und innerhalb der Maschine angeordnetes Trocknungssystem umfasst, welches zwei unterschiedliche lufttechnische Kreisläufe umfasst:
• einen ersten lufttechnischen Kreislauf (A, B, C, L1, D, E, F, L2, G), in welchem die aus der Waschwanne (A) der Endoskop-Waschmaschine (10) stammende Luft als Spülluft in die Kanäle des Endoskops gedrückt wird, um eine innere Spülung der Kanäle des behandelten Endoskops durchzuführen, wobei Wasser entfernt wird, um den Durchgang der Trocknungsluft zu ermöglichen;
• einen zweiten lufttechnischen Kreislauf (I, H, L, M, L3, F, D, G), in welchem die aus der inneren Umgebung der Endoskop-Waschmaschine (10) stammende Luft, die auch mit den durch die Verwendung von Peressigsäure erzeugten Dämpfen kontaminiert sein kann, zuvor durch ein erstes Aktivkohle-Filtermedium (H) gefiltert wird und anschließend in die inneren Kanäle des Endoskops gedrückt wird, um als Trocknungsluft verwendet zu werden und das Trocknen der Kanäle des behandelten Endoskops zu ermöglichen.

2. Endoskop-Waschmaschine (10) nach Anspruch 1, **dadurch gekennzeichnet, dass** der erste lufttechnische Kreislauf umfasst:
• eine Wanne (A) zum Waschen und Spülen eines zu behandelnden Endoskops, die eine Ablauföffnung (B) aufweist;
• eine Pumpe (C), die eine Luftansaugöffnung aufweist, welche mittels erster pneumatischer Leitungsmittel (L1) pneumatisch mit der Ablauföffnung (B) der Wanne (A) verbunden ist und dazu ausgelegt ist, Luft aus der Ablauföffnung (B) anzusaugen, nachdem das Spülwasser aus der Wanne (A) abgeführt worden ist;
• einen Verteiler (D), der einen Lufteinlass aufweist, der pneumatisch mit dem Luftdruckauslass der Pumpe (C) verbunden ist, sowie eine Mehrzahl von Luftauslässen, die stromabwärts des Lufteinlasses angeordnet sind;
• ein Magnetventil (F), welches einen Luftstrom-Umschalter umfasst, der in einer ersten Betriebsstellung den Druckluftstrom vom Lufteinlass des Verteilers (D) zu der Mehrzahl von Luftauslässen des Verteilers (D) leitet;
• einen Drucksensor (E), der dazu ausgelegt ist, den Druck der zwischen dem Lufteinlass und der Mehrzahl von Luftauslässen des Verteilers (D) strömenden Luft in Bezug auf einen voreingestellten Grenzwert zu überwachen;
• eine Mehrzahl von pneumatischen Anschlüssen (G) für entsprechende innere Kanäle des behandelten Endoskops, die am Boden der Wanne (A) vorgesehen sind, wobei jeder Luftauslass der Mehrzahl von Luftauslässen des Verteilers (D) mittels zweiter pneumatischer Leitungsmittel (L2) pneumatisch mit einem jeweiligen pneumatischen Anschluss der Mehrzahl von pneumatischen Anschlüssen (G) verbunden ist,
so dass die aus der Wanne (A) entnommene Spülluft verwendet wird, um eine innere Spülung der Kanäle des behandelten Endoskops durchzuführen, wobei Wasser aus dem inneren Kreislauf des behandelten Endoskops entfernt wird, um in einer nachfolgenden Phase den Durchgang von gefilterter Trocknungsluft zu ermöglichen.

3. Endoskop-Waschmaschine (10) nach Anspruch 1, **dadurch gekennzeichnet, dass** der zweite lufttechnische Kreislauf umfasst:
• eine Gebläseeinrichtung (I) für aus dem inneren Kreislauf der Maschine (10) entnommene Innenluft, die innerhalb der Maschine (10) angeordnet ist, wobei die Innenluft durch die Gebläseeinrichtung (I) durch das erste Aktivkohle-Filtermedium (H) geblasen wird, das innerhalb der Maschine (10) angeordnet ist und dazu ausgelegt ist, aus der Innenluft die toxischen Dämpfe der zur Desinfektion des in der Wanne (A) angeordneten endoskopischen Instruments verwendeten Säure zu entfernen;
• ein zweites Filtermedium (L), das innerhalb der Maschine (10) angeordnet ist, stromabwärts des ersten Filtermediums (H) vorgesehen ist und einen Filtrationsgrad in der Größenordnung von 0,01 µm aufweist, wobei ein Lufteinlass des zweiten Filtermediums (L) die durch das erste Filtermedium (H) gefilterte Innenluft empfängt und diese durch zusätzliche Filtration steril macht;
• eine Mehrzahl von Auslässen für sterile Luft (M) des zweiten Filtermediums (L), die mittels dritter pneumatischer Leitungsmittel (L3) pneumatisch mit jeweiligen Lufteinlässen des Magnetventils (F) verbunden ist, welches in einer zweiten Betriebsstellung des jeweiligen Luftstrom-Umschalters den Druckluftstrom von den jeweiligen Lufteinlässen des Magnetventils (F) zu der Mehrzahl von Luftauslässen des Verteilers (D) leitet, wobei:
• die Mehrzahl von Luftauslässen des Verteilers (D) mittels vierter pneumatischer Leitungsmittel (L4) jeweils pneumatisch mit der Mehrzahl von pneumatischen Anschlüssen (G) für entsprechende innere Kanäle des behandelten Endoskops verbunden ist, das in der Wanne (A) angeordnet ist;
• die gefilterte Innenluft durch den Verteiler (D) über die jeweiligen pneumatischen Anschlüsse (G) in die inneren Kanäle des Endoskops gedrückt wird,
so dass die Endoskop-Waschmaschine (10) das Trocknen der Kanäle des behandelten Endoskops sowie eine Entfernung der aus dem Desinfektionsmittel stammenden Säuredämpfe durchführt.

## Revendications

1. Machine (10) de lavage d'endoscopes pour le séchage des canaux internes des endoscopes reconditionnés, **caractérisée en ce qu'**elle comprend un système de séchage intégré et interne à la machine, comprenant deux circuits aérauliques distincts:
• un premier circuit aéraulique (A, B, C, L1, D, E, F, L2, G), dans lequel l'air provenant de la cuve de lavage (A) de la machine de lavage d'endoscopes (10) est poussé dans les canaux de l'endoscope en tant qu'air de purge, afin d'effectuer une purge interne des canaux de l'endoscope traité, en éliminant l'eau pour permettre le passage de l'air de séchage ;
• un second circuit aéraulique (I, H, L, M, L3, F, D, G), dans lequel l'air provenant de l'environnement interne de la machine de lavage d'endoscopes (10), éventuellement contaminé par les fumées générées par l'utilisation de l'acide peracétique, est préalablement filtré à travers un premier moyen filtrant à charbon actif (H), puis est poussé dans les canaux internes de l'endoscope pour être utilisé comme air de séchage et permettre le séchage des canaux de l'endoscope traité.

2. Machine (10) de lavage d'endoscopes selon la revendication 1, **caractérisée en ce que** ledit premier circuit aéraulique comprend :
• une cuve (A) pour le lavage et le rinçage d'un endoscope à traiter, présentant une ouverture d'évacuation (B) ;
• une pompe (C), présentant une ouverture d'aspiration d'air pneumatiquement reliée, au moyen de premiers moyens de conduit pneumatique (L1), à ladite ouverture d'évacuation (B) de la cuve (A), et configurée pour prélever de l'air depuis ladite ouverture d'évacuation (B), après l'évacuation de l'eau de rinçage de la cuve (A) ;
• un collecteur (D), présentant une entrée d'air pneumatiquement reliée à la sortie de refoulement d'air de ladite pompe (C) et une pluralité de sorties d'air, disposées en aval de ladite entrée d'air ;
• une électrovanne (F), comprenant un déviateur de flux d'air qui, dans une première position opérationnelle, canalise le flux d'air comprimé depuis ladite entrée d'air dudit collecteur (D) vers ladite pluralité de sorties d'air dudit collecteur (D) ;
• un capteur de pression (E), configuré pour contrôler la pression de l'air s'écoulant entre ladite entrée et ladite pluralité de sorties d'air du collecteur (D) par rapport à une valeur limite prédéfinie ;
• une pluralité de raccords pneumatiques (G) pour des canaux internes correspondants de l'endoscope traité, prévus au fond de la cuve (A), dans laquelle chaque sortie d'air de ladite pluralité de sorties d'air dudit collecteur (D) est pneumatiquement reliée, au moyen de seconds moyens de conduit pneumatique (L2), à un raccord pneumatique respectif de ladite pluralité de raccords pneumatiques (G),
de sorte que l'air de purge, prélevé depuis la cuve (A), est utilisé pour effectuer une purge interne des canaux de l'endoscope traité, en éliminant l'eau du circuit interne de l'endoscope traité afin de permettre, dans une phase ultérieure, le passage d'un air de séchage filtré.

3. Machine (10) de lavage d'endoscopes selon la revendication 1, **caractérisée en ce que** ledit second circuit aéraulique comprend :
• un dispositif souffleur (I) d'air interne prélevé depuis le circuit interne de la machine (10), disposé à l'intérieur de la machine (10), dans lequel ledit air interne est soufflé par le dispositif souffleur (I) à travers ledit premier moyen filtrant (H) à charbon actif, disposé à l'intérieur de la machine (10) et configuré pour éliminer de l'air interne les fumées toxiques de l'acide utilisé pour la désinfection de l'instrument endoscopique disposé à l'intérieur de la cuve (A) ;
• un second moyen filtrant (L), disposé à l'intérieur de la machine (10), placé en aval dudit premier moyen filtrant (H) et présentant un degré de filtration de l'ordre de 0,01 µm, dans lequel une entrée d'air dudit second moyen filtrant (L) reçoit l'air interne filtré par ledit premier moyen filtrant (H) et, au moyen d'une filtration supplémentaire, rend l'air interne stérile ;
• une pluralité de sorties d'air stérile (M) dudit second moyen filtrant (L), laquelle est pneumatiquement reliée, au moyen de troisièmes moyens de conduit pneumatique (L3), à des entrées d'air respectives de ladite électrovanne (F) qui, dans une seconde position opérationnelle du déviateur de flux d'air respectif, canalise le flux d'air comprimé depuis lesdites entrées d'air respectives de ladite électrovanne (F) vers ladite pluralité de sorties d'air dudit collecteur (D), dans lequel :
• ladite pluralité de sorties d'air dudit collecteur (D) est pneumatiquement reliée, au moyen de quatrièmes moyens de conduit pneumatique (L4), de manière respective, à ladite pluralité de raccords pneumatiques (G) pour des canaux internes correspondants de l'endoscope traité, disposé dans la cuve (A) ;
• l'air interne filtré est poussé par le collecteur (D), à travers les raccords pneumatiques respectifs (G), dans les canaux internes de l'endoscope,
de sorte que la machine de lavage d'endoscopes (10) effectue le séchage des canaux de l'endoscope traité et une élimination des fumées acides provenant du désinfectant.
